# EUROPEAN PATENT SPECIFICATION

(11) **EP 0 521 820 B1**
(45) Date of publication and mention of the grant of the patent: **07.02.1996**
(21) Application number: 92810399.3
(22) Date of filing: 26.05.1992
(51) Int. Cl.: C07D 211/22, C07D 211/32, C08K 5/3435

(54) **2,6-Diarylpiperidin-1-yl substituted 2-butene stabilizers**
2,6-Diarylpiperidin-1-yl substituierte 2-Butene als Stabilisatoren
2,6-Diarylpipéridine-1-yl-butènes-2 comme stabilisateurs

(30) Priority: 03.06.1991 US 709688
(43) Date of publication of application: 07.01.1993
(73) Proprietor: CIBA-GEIGY AG, CH-4002 Basel (CH)
(72) Inventor: Cunkle, Glen T., Stamford, CT 06906 (US); Babiarz, Joseph E., Amawalk, NY 10501 (US)

(56) References cited:
- EP-A- 0 249 145
- GB-A- 1 438 482
- US-A- 3 932 564
- CHEMICAL ABSTRACTS, vol. 80, no. 21, 27 May 1974, Columbus, Ohio, US; abstract no. 120713H, SHARIFKANOV,A,SH. ET AL.: 'Synthesis of the beta-isomers of 1,4-bis[4-(benzoyloxy)-2,5-dimet hylpiperidyl]-2-butenes.' page 417 ;column 1 ;
- CHEMICAL ABSTRACTS, vol. 76, no. 3, 17 January 1972, Columbus, Ohio, US; abstract no. 14281Y, SHARIFKANOV, A. SH.ET AL.: 'Synthesis of benzoic esters of 1,4-bis(2,5-dimethyl-4-hydroxypiperidi no)-2-butene and -2-butyne.' page 363 ;column 2 ;
- CHEMICAL ABSTRACTS, vol. 77, no. 13, 25 September 1972, Columbus, Ohio, US; abstract no. 87725B, BABAYAN, A. T. ET AL.: 'Amines and ammonium compounds.LXXXVI. Synthesis of unsymmetrical 1,4-ditertiary and secondary-tertiary diamines with a common unsaturated group.' page 392 ;column 2 ;

## Description

The present invention pertains to novel 2,6-diarylpiperidin-1-yl substituted 2-butene compounds and their use as stabilizers for various organic materials subject to the deleterious effects of oxygen, heat or actinic radiation. The instant compounds provide good retention of polymer physical properties during long-term thermooxidative stress.

### Background of the Invention

Substituted 1,4-diamino-2-butene compounds are known in the art where the substitution on the N-atoms is alkyl or benzyl, but their use as stabilizers is not disclosed or suggested.

When such substitution is aryl, British Patent No. 1,438,482 generically describes N,N,N',N'-tetraaryl-2-butene-1,4-diamine as stabilizers for lubricant compositions, but does not specifically disclose such compounds. British 1,438,482 does not suggest that such aryl substituted compounds can provide effective antioxidant protection to synthetic polymers.

The compounds of this invention and their use as stabilizers for organic materials subject to degradation by oxygen, heat or light are not disclosed or suggested in the prior art.

### Objects of the Invention

One object of this invention is to provide new substituted 2-butene compounds which are useful stabilizers for various substrates.

Another object of the invention is to provide synthetic polymer compositions stabilized against the deleterious effects of oxygen, heat and light by incorporating therein an effective amount of an instant compound.

### Detailed Disclosure

The instant invention pertains to novel compounds of formula I or II

T-CH₂-CH=CH-CH₂-T (I)

T-CH₂-CH=CH-CH₂-N(E₁)(E₂) (II)

wherein
E₁ and E₂ are independently alkyl of 1 to 30 carbon atoms, said alkyl terminated with cycloalkyl of 5 to 12 carbon atoms, said alkyl terminated with -CN, -OR₁₆, -NR₁₇R₁₈, -SR₁₉, -COOR₂₀ or -CONR₂₁R₂₂, where R₁₆, R₁₇, R₁₈, R₁₉ and R₂₀ are independently alkyl of 1 to 20 carbon atoms or alkenyl of 3 to 18 carbon atoms, and R₂₁ and R₂₂ are independently hydrogen or the same meaning as R₁₆; or said alkyl interrupted by one or more -O-, -S-, -SO-, -SO₂-,-CO-, -COO-, -OCO-, -CONR₂₃-, -NR₂₃CO- or -NR₂₄- where R₂₃ and R₂₄ have the same meaning as R₂₁; alkenyl of 3 to 20 carbon atoms; aralkyl of 7 to 15 carbon atoms or said aralkyl substituted on the aryl ring by one to three groups selected from alkyl of 1 to 12 carbon atoms, -CN, -NO₂, halogen, -OR₁₆, -NR₁₇R₁₈, -SR₁₉, -COOR₂₀ or -CONR₂₁R₂₂, or aryl of 6 to 10 carbon atoms or said aryl substituted by one to three substituents selected from the group consisting of alkyl of 1 to 20 carbon atoms, cycloalkyl of 5 to 12 carbon atoms and aralkyl of 7 to 15 carbon atoms;
T is a group of formula III, IV, V or VI 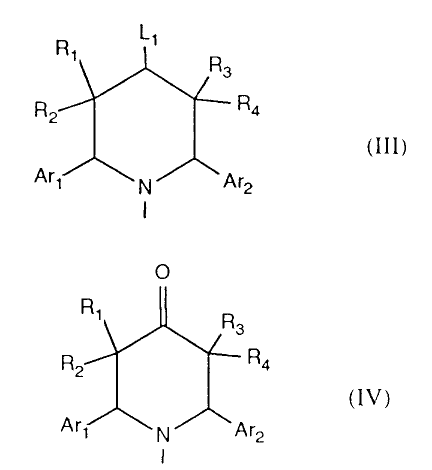 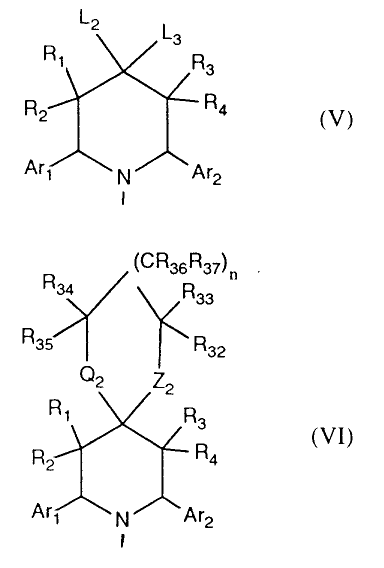
where Ar₁ and Ar₂ are independently aryl of 6 to 10 carbon atoms; or said aryl substituted by one to three substituents selected from the group consisting of alkyl of 1 to 20 carbon atoms, cycloalkyl of 5 to 12 carbon atoms, phenylalkyl of 7 to 15 carbon atoms, -COOG₁ where G₁ is hydrogen or alkyl of 1 to 20 carbon atoms, -COG₂ where G₂ is alkyl of 1 to 20 carbon atoms, -NR₅R₆ where R₅ and R₆ are independently hydrogen or alkyl of 1 to 20 carbon atoms -SG₃ where G₃ is aryl of 6 to 10 carbon atoms or alkyl of 1 to 20 carbon atoms, -OH, -OCH₃, -CN, -CF₃, -NO₂, -F, -Cl, -Br or -I;
R₁, R₂, R₃ and R₄ are independently hydrogen; a linear or branched alkyl of 1 to 30 carbon atoms; said alkyl terminated with -OR₇, -NR₈R₉, -SR₁₀, -COOR₁₁ or -CONR₁₂R₁₃, where R₇, R₈, R₉, R₁₀ and R₁₁ are independently hydrogen, alkyl of 1 to 20 carbon atoms or alkenyl of 3 to 18 carbon atoms, and R₁₂ and R₁₃ are independently hydrogen or the same meaning as R₁₁; or said alkyl interrupted by one or more -O-, -S-, -SO-, -SO₂-, -CO-, -COO-, -OCO-, -CONR₁₄-, -NR₁₄CO- or -NR₁₅- where R₁₄ and R₁₅ have the same meaning as R₁₂; alkenyl of 3 to 20 carbon atoms; or aryl of 6 to 10 carbon atoms;
L₁ is hydrogen, -OR₂₅ where R₂₅ is hydrogen or alkyl of 1 to 30 carbon atoms, -NR₂₆R₂₇ , -OCOR₂₈, -NCOR₂₉ where R₂₆, R₂₇, R₂₈ and R₂₉ have the same meaning as R₂₅;
L₂ and L₃ are independently -Q₁-R₃₀ or -Z₁-R₃₁ where Q₁ and Z₁ are independently -O-, -S- or -NR₂₆-, and R₃₀ and R₃₁ are hydrogen or alkyl of 1 to 30 carbon atoms; and
n is 0 or 1, Q, and Z₂ are independently -O-, -S- or -NR₂₆-, and R₃₂, R₃₃, R₃₄, R₃₅, R₃₆ and R₃₇ are independently hydrogen or alkyl of 1 to 30 carbon atoms.

In the above formulae and throughout the specification and claims the indices of chemical symbols denote the number of the respective atoms. In contrast, the indices of symbols which have no chemical meaning, such as R or L, together with the symbol denote a freely defined radical.

Preferably E₁ and E₂ are independently alkyl of 4 to 20 carbon atoms or said alkyl interrupted by one or more -O-, -S-,-CO-, -COO-, -OCO-, -CONR₂₃-, -NR₂₃CO- or -NR₂₄-; alkenyl of 3 to 12 carbon atoms; phenylalkyl of 7 to 15 carbon atoms or said phenylalkyl substituted on the phenyl ring by one to three groups selected from alkyl of 1 to 12 carbon atoms, -OR₁₆, -NR₁₇R₁₈, -COOR₂₀ or -CONR₂₁R₂₂; or phenyl or said phenyl substituted by one to three substituents selected from the group consisting of alkyl of 1 to 20 carbon atoms and cycloalkyl of 5 to 7 carbon atoms. Most preferably E₁ and E₂ are independently alkyl of 4 to 20 carbon atoms, phenylalkyl of 7 to 15 carbon atoms or said phenylalkyl substituted on the phenyl ring by one or two alkyl groups of 1 to 4 carbon atoms and/or -OH, or phenyl, tolyl or xylyl; especially preferred are E₁ and E₂ independently alkyl of 4 to 20 carbon atoms or benzyl.

Preferred are compounds of formula II.

Preferably T is a group of formula III or IV; most preferably T is a structure of formula III where L₁ is hydrogen.

Preferably L₁ is hydrogen or -OH.

Preferably L₂ and L₃ are independently -O-R₃₀ or -S-R₃₁ where not more than one of R₃₀ and R₃₁ are hydrogen.

Preferably Ar₁ and Ar₂ are independently phenyl; or said phenyl substituted by one to three substituents selected from the group consisting of alkyl of 1 to 20 carbon atoms, cycloalkyl of 5 to 7 carbon atoms, phenylalkyl of 7 to 15 carbon atoms, -OH, -OCH₃, -COOG₁, -COG₂ or -NR₅R₆. Most preferably Ar₁ and Ar₂ are independently phenyl; or said phenyl substituted by cyclohexyl, -OH, -OCH₃ and/or one or two alkyl groups of 1 to 4 carbon atoms. Especially preferred is a compound wherein Ar₁ and Ar₂ are phenyl or phenyl substituted by one or two methyl groups.

Preferably R₁, R₂, R₃ and R₄ are independently hydrogen, methyl, ethyl, -CH₂-OH, -CH₂-NH₂, -C₂H₄-OH, -C₂H₄-NH₂, allyl or phenyl; most preferably hydrogen or methyl.

A preferred embodiment of this invention therefore is a compound wherein E₁ and E₂ are independently alkyl of 4 to 20 carbon atoms or said alkyl interrupted by one or more -O-, -S-, -CO-, -COO-, -OCO-, -CONR₂₃-, -NR₂₃CO- or -NR₂₄- where R₂₃ and R₂₄ are as defined above; alkenyl of 3 to 12 carbon atoms; phenylalkyl of 7 to 15 carbon atoms or said phenylalkyl substituted on the phenyl ring by one to three groups selected from alkyl of 1 to 12 carbon atoms, -OR₁₆, -NR₁₇R₁₈, -COOR₂₀ or -CONR₂₁R₂₂; or phenyl or said phenyl substituted by one to three substituents selected from the group consisting of alkyl of 1 to 20 carbon atoms and cycloalkyl of 5 to 7 carbon atoms;
T is a group of formula III or IV;
Ar₁ and Ar₂ are independently phenyl; or said phenyl substituted by one to three substituents selected from the group consisting of alkyl of 1 to 20 carbon atoms, cycloalkyl of 5 to 7 carbon atoms, phenylalkyl of 7 to 15 carbon atoms, -OH, -OCH₃, -COOG₁, -COG₂ or -NR₅R₆, where G₁, G₂, R₅ and R₆ are as defined above;
R₁, R₂, R₃ and R₄ are independently hydrogen, methyl, ethyl, -CH₂-OH, -CH₂-NH₂, -C₂H₄-OH, -C₂H₄-NH₂, allyl or phenyl; and
L₁ is as defined above.

Especially preferred is a compound wherein
E₁ and E₂ are independently alkyl of 4 to 20 carbon atoms; phenylalkyl of 7 to 15 carbon atoms; or said phenylalkyl substituted on the phenyl ring by one or two alkyl groups of 1 to 4 carbon atoms and/or -OH; or phenyl, tolyl or xylyl;
Ar₁ and Ar₂ are independently phenyl; or said phenyl substituted by cyclohexyl, -OH, -OCH₃ and/or one or two alkyl groups of 1 to 4 carbon atoms; and
L₁ is hydrogen or -OH.

The instant invention also pertains to a composition stabilized against the deleterious effects of oxygen, heat or actinic light which comprises
(a) an organic material subject to oxidative, thermal or actinic degradation, and
(b) a compound of formula I or II as defined above.

Appropriately the compositions according to the invention comprise an amount of at least one compound of formula I or II which is effective for stabilizing the organic material.

The preparation of 2,6-diphenylpiperidine is described by V. Balaih et al., Indian J. Chem., 16B, 1065 (1978).

The compounds of formula I and formula II are conveniently prepared by reacting an appropriate secondary amine such as 2,6-diphenylpiperidine with 1,4-but-2-enediol diacetate in the presence of a catalyst such as tetrakis(triphenylphosphine)palladium(0) in an organic solvent such as tetrahydrofuran.

The starting materials for making the instant compounds are largely items of commerce.

When any of Ar₁,Ar₂, R₁ to R₃₇, E₁, E₂, L₁, L₂, or G₁ to G₃ is alkyl, such alkyl groups are, for example, methyl, ethyl, isopropyl, n-butyl, tert-butyl, tert-amyl, 2-ethylhexyl, n-octyl, n-undecyl, lauryl, n-heptadecyl, n-octadecyl, eicosyl and tricontyl; when said radicals are cycloalkyl, they are, for example, cyclopentyl, cyclohexyl, cyclooctyl and cyclododecyl; when said radicals are aralkyl, they are, for example, benzyl, phenethyl, a-methylbenzyl, a,a-dimethylbenzyl and 1-naphthylmethyl; when said radicals are aryl, they are, for example, phenyl, naphthyl or when substituted by alkyl are, for example, tolyl and xylyl; and when said radicals are alkyl interrupted by -O-, they are, for example, 3-oxaamyl and 3,6-dioxaoctyl.

The compositions where component (a) is a synthetic polymer are especially a part of this invention, most particularly when the synthetic polymer is a polyolefin such as polypropylene or is an elastomer such as dynamically crosslinked polypropylene/nitrile rubber.

The instant compounds are effective stabilizers for synthetic polymers subject to the deleterious effects of heat and/or oxygen especially during processing at elevated temperatures.

The invention therefore also pertains to a process for stabilizing an organic material against oxidative, thermal or actinic degradation, which process comprises incorporating in said material at least one compound of formula I or II. This includes the use of said compounds as stabilizer for protecting organic material against oxidative, thermal or actinic degradation, especially the use for protecting a polyolefin or an elastomer against degradation during processing at elevated temperatures.

In general polymers which can be stabilized include
1. Polymers of monoolefins and diolefins, for example polypropylene, polyisobutylene, polybut-1-ene, polymethylpent-1-ene, polyisoprene or polybutadiene, as well as polymers of cycloolefins, for example of cyclopentene or norbornene, polyethylene (which may be crosslinked), for example high density polyethylene (HDPE), low density polyethylene (LDPE), linear low density polyethylene (LLDPE), branched low density polyethylene (BLDPE).
2. Mixtures of the polymers mentioned in 1), for example mixtures of polypropylene with polyisobutylene, polypropylene with polyethylene (for example PP/HDPE, PP/LDPE) and mixtures of different types of polyethylene (for example LDPE/HDPE).
3. Copolymers of monoolefins and diolefins with each other or with other vinyl monomers, for example ethylene/propylene, linear low density polyethylene (LLDPE) and its mixtures with low density polyethylene (LDPE), propylene/butene-1, ethylene/hexene, ethylene/ethylpentene, ethylene/heptene, ethylene/octene, propylene/isobutylene, ethylene/but-1-ene, propylene/butadiene, isobutylene/isoprene, ethylene/alkyl acrylates, ethylene/alkyl methacrylates, ethylene/vinyl acetate copolymers and their copolymers with carbon monoxide or ethylene/acrylic acid copolymers and their salts (ionomers) and terpolymers of ethylene with propylene and a diene, such as hexadiene dicyclopentadiene or ethylidene-norbornene; as well as mixtures of such copolymers and their mixtures with polymers mentioned in 1) above, for example
   polypropylene/ethylene-propylene-copolymers, LDPE/ethylene-vinyl acetate copolymers (EVA), LDPE/ethylene-acrylic acid copolymers (EAA), LLDPE/EVA, LLDPE/EAA and random or alternating polyalkylene/carbon monoxide-copolymers as well as mixtures thereof with other polymers, for example polyamides.
3a. Hydrocarbon resins (for example C₅-C₉) and hydrogenated modifications thereof (for example tackifiers) and mixtures of polyalkylenes and starch.
4. Polystyrene, poly(p-methylstyrene), poly(α-methylstyrene).
5. Copolymers of styrene or α-methylstyrene with dienes or acrylic derivatives, such as, for example, styrene/butadiene, styrene/acrylonitrile, styrene/alkyl methacrylate, styrene/maleic anhydride, styrene/butadiene/alkyl acrylate, styrene/butadiene/alkyl methacrylate styrene/acrylonitrile/methyl acrylate; mixtures of high impact strength from styrene copolymers and another polymer, for example from a polyacrylate, a diene polymer or an ethylene/propylene/diene terpolymer; and block copolymers of styrene, for example styrene/butadiene/styrene, styrene/isoprene/styrene, styrene/ethylene/butylene/styrene or styrene/ethylene/propylene/ styrene.
6. Graft copolymers of styrene or α-methylstyrene, for example styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed in 5), for example the copolymer mixtures known as ABS, MBS, ASA or AES polymers.
7. Halogen-containing polymers such as polychloroprene, chlorinated rubbers, chlorinated or sulfochlorinated polyethylene, copolymers of ethylene and chlorinated ethylene, epichlorohydrin homo- and copolymers, polymers of halogenated vinyl compounds, for example polyvinyl chloride, polyvinylidene chloride, polyvinyl fluoride, polyvinylidene fluoride, as well as copolymers thereof, for example vinyl chloride/vinylidene chloride, vinyl chloride/vinyl acetate or vinylidene chloride/vinyl acetate copolymers.
8. Polymers derived from α,β-unsaturated acids and derivatives thereof, such as polyacrylates and polymethacrylates, polymethyl methacrylate impact-modified with butyl acrylate, polyacrylamides and polyacrylonitriles.
9. Copolymers of the monomers mentioned in 8) with each other or with other unsaturated monomers, for example acrylonitrile/ butadiene, acrylonitrile/alkyl acrylate, acrylonitrile/alkoxyalkyl acrylate or acrylonitrile/vinyl halide copolymers or acylonitrile/alkyl methacrylate/butadiene terpolymers.
10. Polymers derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl butyral, polyallyl phthalate or polyallylmelamine; as well as their copolymers with olefins mentioned in 1) above.
11. Homopolymers and copolymers of cyclic ethers, such as polyalkylene glycols, polyethylene oxide, polypropylene oxide or copolymers thereof with bisglycidyl ethers.
12. Polyacetals, such as polyoxymethylene and those polyoxymethylenes which contain ethylene oxide as comonomer; polyacetals modified with thermoplastic polyurethanes, acrylates or MBS.
13. Polyphenylene oxides and sulfides, and mixtures of polyphenylene oxides with polystyrene or polyamides.
14. Polyurethanes derived from polyethers, polyesters or polybutadienes with terminal hydroxyl groups on the one hand and aliphatic or aromatic polyisocyanates on the other, as well as precursors thereof (polyisocyanates, polyols or prepolymers).
15. Polyamides and copolyamides derived from diamines and dicarboxylic acids and/or from aminocarboxylic acids or the corresponding lactams, such as polyamide 4, polyamide 6, polyamide 6/6, 6/10, 6/9, 6/12, 4/6, 12/12, polyamide 11, polyamide 12, aromatic polyamides obtained by condensation of m-xylene, diamine and adipic acid; polyamides prepared from hexamethylenediamine and isophthalic acid and/or terephthalic acid and optionally an elastomer as modifier, for example poly(2,4,4,-trimethylhexamethylene) terephthalamide or poly-m-phenylene isophthalamide. Further copolymers of the aforementioned polyamides with polyolefins, olefin copolymers, ionomers or chemically bonded or grafted elastomers; or with polyethers, as with polyethylene glycols, polypropylene glycols or polytetramethylene glycols. Polyamides or copolyamides modified with EPDM or ABS. Polyamides condensed during processing (RIM polyamide systems).
16. Polyureas, polyimides and polyamide-imides.
17. Polyesters derived from dicarboxylic acids and diols and/or from hydroxycarboxylic acids or the corresponding lactones, such as polethylene terephthalate, polybutylene terephthalate, poly-1,4-dimethylolcyclohexane terephthalate, poly[2,2-(4-hydroxyphenyl)propane] terephthalate and polyhydroxybenzoates as well as block-copolyether-esters derived from polyethers having hydroxyl end groups.
18. Polycarbonates and polyester carbonates.
19. Polysulfones, polyether sulfones and polyether ketones.
20. Crosslinked polymers which are derived from aldehydes on the one hand and phenols, ureas and melamines on the other, such as phenol/formaldehyde resins, urea/formaldehyde resins and melamine/formaldehyde resins.
21. Drying and non-drying alkyd resins.
22. Unsaturated polyester resins derived from copolyesters of saturated and unsaturated dicarboxylic acids with polyhydric alcohols and vinyl compounds as crosslinking agents, and also halogenated modifications thereof of low inflammability.
23. Thermosetting acrylic resins derived from substituted acrylic esters, such as epoxy acrylates, urethane acrylates or polyester acrylates.
24. Alkyd resins, polyester resins or acrylate resins in admixture with melamine resins, urea resins, polyisocyanates or epoxy resins as crosslinking agents.
25. Crosslinked epoxy resins which are derived from polyepoxides, for example from bisglycidyl ethers or from cycloaliphatic diepoxides.
26. Natural polymers, such as cellulose, rubber, gelatin and chemically modified homologous derivatives thereof, such as cellulose acetates, cellulose propionates and cellulose butyrates, or the cellulose ethers, such as methyl cellulose; rosins and their derivatives.
27. Mixtures of polymers as mentioned above, for example PP/EPDM, polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.
28. Naturally occurring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellithates) and also mixtures of synthetic esters with mineral oils in any weight ratios, which materials may be used as plasticizer for polymers or as textile spinning oils, as well as aqueous emulsions of such materials.
29. Aqueous emulsions of natural or synthetic rubber, e.g. natural latex or latices of carboxylated styrene/butadiene copolymers.
30. Polysiloxanes such as the soft, hydrophilic polysiloxanes described, for example, in U.S. Patent No. 4,259,467; and the hard polyorganosiloxanes described, for example, in U.S. Patent No. 4,355,147.
31. Polyketimines in combination with unsaturated acrylic polyacetoacetate resins or with unsaturated acrylic resins. The unsaturated acrylic resins include the urethane acrylates, polyether acrylates, vinyl or acryl copolymers with pendant unsaturated groups and the acrylated melamines. The polyketimines are prepared from polyamines and ketones in the presence of an acid catalyst.
32. Radiation curable compositions containing ethylenically unsaturated monomers or oligomers and a polyunsaturated aliphatic oligomer.
33. Epoxymelamine resins such as light-stable epoxy resins crosslinked by an epoxy functional coetherified high solids melamine resin such as LSE-4103 (Monsanto).

In general, the compounds of the present invention are employed in from about 0.01 to about 5% by weight of the stabilized composition, although this will vary with the particular substrate and application. An advantageous range is from about 0.05 to about 2%, and especially 0.1 to about 1%.

The stabilizers of the instant invention may readily be incorporated into the organic polymers by conventional techniques, at any convenient stage prior to the manufacture of shaped articles therefrom. For example, the stabilizer may be mixed with the polymer in dry powder from, or a suspension or emulsion of the stabilizer may be mixed with a solution, suspension, or emulsion of the polymer. The resulting stabilized polymer compositions of the invention may optionally also contain from about 0.01 to about 5%, preferably from about 0.025 to about 2%, and especially from about 0.1 to about 1% by weight of various conventional additives or mixtures thereof. The invention therefore includes compositions comprising in addition to components (a) and (b) one or more conventional stabilizers or conventional additives, such as the materials listed below.
1. Antioxidants
   1.1. Alkylated monophenols, for example 2,6-di-tert-butyl-4-methyl-phenol, 2-tert-butyl-4,6-dimethylphenol, 2,6-di-tert-butyl-4-ethylphenol, 2,6-di-tert-butyl-4-n-butylphenol, 2,6-di-tert-butyl-4-iso-butylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexyl-phenol, 2,6-di-tert-butyl-4-methoxymethylphenol, 2,6-di-nonyl-4-methylphenol, 2,4-dimethyl-6-(1'-methyl-undec-1'-yl)-phenol, 2,4-dimethyl-6-(1'-methyl-heptadec-1'-yl)-phenol, 2,4-dimethyl-6-(1'-methyl-tridec-1'-yl)-phenol and mixtures therof.
   1.2. Alkylthiomethylphenols, for example 2,4-dioctylthiomethyl-6-tert-butylphenol, 2,4-dioctylthiomethyl-6-methylphenol, 2,4-dioctylthiomethyl-6-ethylphenol, 2,6-di-dodecylthiomethyl-4-nonylphenol.
   1.3. Hydroquinones and alkylated hydroquinones, for example 2,6-di-tert-butyl-4-methoxy-phenol, 2,5-di-tert-butylhydroquinone, 2,5-di-tert-amylhydroquinone, 2,6-diphenyl-4-octadecyloxyphenol, 2,6-di-tert-butyl-hydroquinone, 2,5-di-tert-butyl-4-hydroxyanisole, 3,5-di-tert-butyl-4-hydroxyanisole, 3,5-di-tert-butyl-4-hydroxyphenyl-stearate, bis-(3,5-di-tert-butyl-4-hydroxyphenyl)adipate.
   1.4. Hydroxylated thiodiphenyl ethers, for example 2,2'-thiobis(6-tert-butyl-4-methylphenol), 2,2'-thiobis(4-octylphenol), 4,4'-thiobis(6-tert-butyl-3-methylphenol), 4,4'-thiobis(6-tert-butyl-2-methylphenol), 4,4'-thio-bis-(3,6-di-sec-amylphenol), 4,4'-bis-(2,6-dimethyl-4-hydroxyphenyl)-disulfide.
   1.5. Alkylidenebisphenols, for example 2,2'-methylenebis(6-tert-butyl-4-methylphenol), 2,2'-methylenebis(6-tert-butyl-4-ethylphenol), 2,2'-methylenebis[4-methyl]-6-(α-methylcyclohexyl)phenol], 2,2'-methylenebis(4-methyl-6-cyclohexylphenol), 2,2'-methylenebis(6-nonyl-4-methylphenol), 2,2'-methylenebis(4,6-di-tert-butylphenol), 2,2'-ethylidenebis(4,6-di-tert-butylphenol), 2,2'-ethylidenebis(6-tert-butyl-4-isobutylphenol), 2,2'-methylenebis[6-(α-methylbenzyl)-4-nonylphenol], 2,2'-methylenebis[6-(α,α-dimethylbenzyl)-4-nonylphenol], 4,4'-methylenebis(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methyl-phenyl)-3-n-dodecylmercaptobutane, ethylene glycol bis[3,3-bis-(3'-tert-butyl-4'-hydroxyphenyl)butyrate], bis(3-tert-butyl-4-hydroxy-5-methylphenyl)dicyclopentadiene, bis[2-(3'-tert-butyl-2'-hydroxy-5'-methylbenzyl)-6-tert-butyl-4-methylphenyl]terephthalate, 1,1-bis-(3,5-dimethyl-2-hydroxyphenyl)butan, 2,2-bis-(3,5-di-tert.-butyl-4-hydroxyphenyl)-propan, 2,2-bis-(5-tert.-butyl-4-hydroxy-2-methylphenyl)-4-n-dodecylmercapto-butan, 1,1,5,5-tetra-(5-tert.-butyl-4-hydroxy-2-methylphenyl)-pentan.
   1.6. O-, N- and S-benzyl compounds, for example 3,5,3',5'-tetra-tert.-butyl-4,4'-dihydroxydibenzylether, octadecyl-4-hydroxy-3,5-dimethylbenzyl-mercaptoacetate, tris-(3,5-di-tert.-butyl-4-hydroxybenzyl)-amine, bis-(4-tert.-butyl-3-hydroxy-2,6-dimethylbenzyl)-dithioterephthalate, bis-(3,5-di-tert.-butyl-4-hydroxybenzyl)-sulfide, isooctyl-3,5-di-tert.-butyl-4-hydroxybenzyl-mercaptoacetate.
   1.7. Hydroxybenzylated Malonates, for example
      dioctadecyl-2,2-bis-(3,5-di-tert.-butyl-2-hydroxybenzyl)-malonate, di-octadecyl-2-(3-tert.-butyl-4-hydroxy-5-methylbenzyl)-malonate, di-dodecylmercaptoethyl-2,2-bis-(3,5-di-tert.-butyl-4-hydroxybenzyl)-malonate, Di-[4-(1,1,3,3-tetramethylbutyl)-phenyl]-2,2-bis-(3,5-di-tert.-butyl-4-hydroxybenzyl)-malonate.
   1.8. Hydroxybenzyl-Aromatics, for example 1,3,5-tris-(3,5-di-tert.-butyl-4-hydroxybenzyl)-2,4,6-trimethylbenzene, 1,4-bis-(3,5-di-tert.-butyl-4-hydroxybenzyl)-2,3,5,6-tetramethylbenzene, 2,4,6-tris-(3,5-di-tert.-butyl-4-hydroxybenzyl)-phenol.
   1.9. Triazine Compounds, for example
      2,4-bis-octylmercapto-6-(3,5-di-tert.-butyl-4-hydroxyanilino)-1,3,5-triazine, 2-octylmercapto-4,6-bis-(3,5-di-tert.-butyl-4-hydroxyanilino)-1,3,5-triazine, 2-octylmercapto-4,6-bis-(3,5-di-tert.-butyl-4-hydroxyphenoxy)-1,3,5-triazine, 2,4,6-tris-(3,5-di-tert.-butyl-4-hydroxyphenoxy)-1,2,3-triazine, 1,3,5-tris-(3,5-di-tert.-butyl-4-hydroxybenzyl)-isocyanurate, 1,3,5-tris-(4-tert.-butyl-3-hydroxy-2,6-dimethylbenzyl)-isocyanurate, 2,4,6-tris-(3,5-di-tert.-butyl-4-hydroxyphenylethyl)-1,3,5-triazine, 1,3,5-tris-(3,5-di-tert.butyl-4-hydroxyphenylpropionyl)-hexahydro-1,3,5-triazine, 1,3,5-tris-(3,5-dicyclohexyl-4-hydroxybenzyl)-isocyanurate.
   1.10. Benzylphosphonates, for example dimethyl-2,5-di-tert.-butyl-4-hydroxybenzylphosphonate, diethyl-3,5-di-tert.-butyl-4-hydroxybenzylphosphonate, dioctadecyl-3,5-di-tert.-butyl-4-hydroxybenzylphosphonate, dioctadecyl-5-tert.-butyl-4-hydroxy-3-methylbenzylphosphonate, Ca-salt of the 3,5-di-tert.-butyl-4-hydroxybenzyl-phosphonic acid monoethylester.
   1.11. Acylaminophenols, for example lauric acid 4-hydroxyanilide, stearic acid 4-hydroxyanilide, octyl N-(3,5-di-tert-butyl-4-hydroxyphenyl)-carbamate.
   1.12. Esters of β-(3,5-di-tert-butyl-4-hydroxyphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N'-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
   1.13. Esters of β-(5-tert-butyl-4-hydroxy-3-methylphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N'-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
   1.14 Esters of β-(3,5-dicyclohexyl-4-hydroxyphenyl)propionic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N'-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
   1.15 Esters of 3,5-di-tert.-butyl-4-hydroxyphenyl acetic acid with mono- or polyhydric alcohols, e.g. with methanol, ethanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N'-bis(hydroxyethyl)oxalic acid diamide, 3-thiaundecanol, 3-thiapentadecanol, trimethylhexanediol, trimethylolpropane, 4-hydroxymethyl-1-phospha-2,6,7-trioxabicyclo-[2.2.2]-octane.
   1.16. Amides of β-(3,5-di-tert-butyl-4-hydroxyphenyl)propionic acid e.g. N,N'-bis(3,5-di-tert-butyl-4-hydroxyphenylpropionyl)hexamethylene-diamine, N,N'-bis(3,5-di-tert-butyl-4-hydroxyphenylpropionyl)tri-methylene-diamine, N,N'-bis(3,5-di-tert-butyl-4-hydroxyphenylpropionyl)-hydrazine.
2. UV absorbers and light stabilisers
   2.1. 2-(2'-Hydroxyphenyl)benzotriazoles, for example the 5'-methyl, 3',5'-di-tert-butyl, 5'-tert-butyl, 5'-(1,1,3,3-tetramethylbutyl), 5-chloro-3',5'-di-tert-butyl, 5-chloro-3'-tert-butyl-5'-methyl, 3'-sec-butyl-5'-tert-butyl, 4'-octoxy, 3',5'-di-tert-amyl and 3',5'-bis(α,α-dimethylbenzyl), mixture of 5-chloro-3'-tert.-butyl-5'-(2-octyloxycarbonylethyl)- and 5-chloro-3'-tert.-butyl-5'-[2-(2-ethylhexyloxy)-carbonylethyl]-, 5-chloro-3'-tert.-butyl-5'-(2-methoxycarbonylethyl)-, 3'-tert.-butyl-5'-(2-methoxycarbonylethyl)-, 3'-tert.-butyl-5'-(2-octyloxycarbonylethyl)-, 3'-tert.-butyl-5'-[2-(2-ethylhexyloxy)-carbonylethyl]-, 3'-dodecyl-5'-methyl- and 3'-tert.-butyl-5'-(2-isooctyloxycarbonylethyl)-2'-hydroxyphenyl-2H-benztriazole(2), 2,2'-methylene-bis[4-(1,1,3,3-tetramethylbutyl)-6-benztriazole-2-yl-phenol]; product of ester interchange of 2-[3'-tert.-butyl-5'-(2-methoxycarbonylethyl)-2'-hydroxy-phenyl]-2H-benztriazole with polyethylene glycol 300; 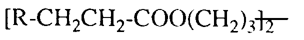 with R=3'-tert.-butyl-4'-hydroxy-5'-2H-benzotriazole-2-yl-phenyl.
   2.2. 2-Hydroxybenzophenones, for example the 4-hydroxy, 4-methoxy, 4-octoxy, 4-decyloxy, 4-dodecyloxy, 4-benzyloxy, 4,2',4'-trihydroxy and 2'-hydroxy-4,4'-dimethoxy derivatives.
   2.3. Esters of substituted and unsubstituted benzoic acids, as for example 4-tert.butylphenyl salicylate, phenyl salicylate, octylphenyl salicylate, dibenzoylresorcinol, bis-(4-tert.butylbenzoyl)-resorcinol, benzoylresorcinol, 2,4-di-tert.butylphenyl 3,5-di-tert.butyl-4-hydroxybenzoate, hexadecyl 3,5-di-tert.butyl-4-hydroxybenzoate, octadecyl 3,5-di-tert.-butyl-4-hydroxybenzoate, 2 methyl-4,6-di-tert.-butylphenyl 3,5-di-tert.-butyl-4-hydroxybenzoate.
   2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxy-cinnamate, butyl α-cyano-β-methyl-p-methoxy-cinnamate, methyl α-carbomethoxy-p-methoxycinnamate and N-(β-carbomethoxy-β-cyanovinyl)-2-methylindoline.
   2.5. Nickel compounds, for example nickel complexes of 2,2'-thio-bis-[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of 4-hydroxy-3,5-di-tert-butylbenzyl-phosphonic acid monoalkyl esters, e.g. of the methyl or ethyl ester, nickel complexes of ketoximes, e.g. of 2-hydroxy-4-methyl-phenyl undecyl ketoxime, nickel complexes of 1-phenyl-4-lauroyl-5-hydroxypyrazole, with or without additional ligands.
   2.6. Sterically hindered amines, for example bis(2,2,6,6-tetramethyl-piperidyl) sebacate, bis-(2,2,6,6-tetramethyl-piperidyl) succinate, bis(1,2,2,6,6-pentamethylpiperidyl) sebacate, bis(1,2,2,6,6-pentamethylpiperidyl) n-butyl-3,5-di-tert-butyl-4-hydroxy-benzylmalonate, the condensation product of 1-(2-hydroxyethyl)-2,2,6,6-tetramethyl-4-hydroxypiperidine and succinic acid, the condensation product of N,N'-bis(2,2,6,6-tetramethyl-4-piperidyl)-hexamethylenediamine and 4-tert-octylamino-2,6-dichloro-1,3,5-triazine, tris(2,2,6,6-tetramethyl-4-piperidyl) nitrilotriacetate, tetrakis(2,2,6,6-tetramethyl-4- piperidyl)-1,2,3,4-butane-tetracarboxylate, 1,1'-(1,2-ethanediyl)bis-(3,3,5,5-tetramethylpiperazinone), 4-benzoyl-2,2,6,6-tetramethylpiperidine, 4-stearyloxy-2,2,6,6-tetramethylpiperidine, bis-(1,2,2,6,6-pentamethylpiperidyl)-2-n-butyl-2-(2-hydroxy-3,5-di-tert.-butylbenzyl) malonate, 3-n-octyl-7,7,9,9-tetramethyl-1,3,8-triazasprio[4.5]decan-2,4-dion, bis-(1-octyloxy-2,2,6,6-tetramethylpiperidyl) sebacate, bis-(1-octyloxy-2,2,6,6-tetramethylpiperidyl) succinate, product of condensation of N,N'-bis-(2,2,6,6-tetramethyl-4-piperidyl)-hexamethylene diamine and 4-morpholino-2,6-dichloro-1,3,5-triazine, product of condensation of-chloro-4,6-di-(4-n-butylamino-2,2,6,6-tetramethylpiperidyl)-1,3,5-triazine and 1,2-bis-(3-aminopropylamino)ethane, product of condensation of 2-chloro-4,6-di-(4-n-butylamino-1,2,2,6,6-pentamethylpiperidyl)-1,3,5-triazine and 1,2-bis-(3-aminopropylamino)ethane, 8-acetyl-3-dodecyl-7,7,9,9-tetramethyl-1,3,8-triazaspiro[4.5]decane-2,4-dion, 3-dodecyl-1-(2,2,6,6-tetramethyl-4-piperidyl)pyrrolidin-2,5-dion, 3-dodecyl-1-(1,2,2,6,6-pentamethyl-4-piperidyl)-pyrrolidin-2,5-dion.
   2.7. Oxalic acid diamides, for example 4,4'-dioctyloxyoxanilide, 2,2'-dioctyloxy-5,5'-di-tert-butyloxanilide, 2,2'-didodecyloxy-5,5'-di-tert-butyloxanilide, 2-ethoxy-2'-ethyloxanilide, N,N'-bis(3-dimethylamino-propyl)oxalamide, 2-ethoxy-5-tert-butyl-2'-ethyloxanilide and its mixture with 2-ethoxy-2'-ethyl-5,4'-di-tert-butyloxanilide and mixtures of ortho- and para-methoxy-disubstituted oxanilides and mixtures of o- and p-ethoxy-disubstituted oxanilides.
   2.8. 2-(2-Hydroxyphenyl)-1,3,5-triazines, for example 2,4,6-tris(2-hydroxy-4-octyloxyphenyl)-1,3,5-triazine, 2-(2-hydroxy-4-octyloxy-phenyl)-4,6-bis(2,4-dimethylphenyl)-1,3,5-triazine, 2-(2,4-dihydroxy-phenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5- triazine, 2-(2-hydroxy-4-dodecyloxyphenyl)-4,6-bis(2,4-dimethylphenyl)-1,3,5-triazine, 2-[2-hydroxy-4-(2-hydroxy-3-butyloxy-propyloxy)phenyl]-4,6-bis(2,4-dimethyl)-1,3,5-triazine, 2-[2-hydroxy-4-(2-hydroxy-3-octyloxy-propyloxy)phenyl]-4,6-bis(2,4-dimethyl)-1,3,5-triazine.
3. Metal deactivators, for example N,N'-diphenyloxalic acid diamide, N-salicylal-N'-salicyloylhydrazine, N,N'-bis(salicyloyl)hydrazine, N,N'-bis(3,5-di-tert-butyl-4-hydroxyphenylpropionyl)hydrazine , 3-salicyl-oylamino-1,2,4-triazole, bis(benzylidene)oxalodihydrazide, Oxanilide, isophthalic acid dihydrazide sebacic acid-bis-phenylhydrazide, N,N'-diacetal-adipinic acid dihydrazide, N,N'-bis-salicyloyl-oxalic acid dihydrazide, N,N'-bis-salicyloyl-thiopropionic acid dihydrazide.
4. Phosphites and phosphonites, for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diiso- decyl pentaerythritol diphosphite, bis(2,4-di-tert.-butylphenyl) pentaerythritol diphosphite, bis-(2,6-di-tert.-butyl-4-methylphenyl)-pentaeryt hritol diphosphite, bis-isodecyloxy-pentaerythritol diphosphite, bis-(2,4-di-tert.-butyl-6-methylphenyl)-pentaerythritol diphosphite, bis-(2,4,6-tri-tert.-butylphenyl)-pentaerythritol diphsophite, tristearyl sorbitol triphosphite, tetrakis(2,4-di-tert-butylphenyl) 4,4'-biphenylene diphosphonite, 6-isooctyloxy-2,4,8,10-tetra-tert.-butyl-12H-dibenz[d,g]-1,3,2-dioxaphosphocin, 6-fluoro-2,4,8,10-tetra-tert.-butyl-12-methyl-dibenz[d,g]-1,3,2-dioxaphosphocin.
5. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(β-dodecyl-mercapto)propionate.
6. Hydroxylamines, for example, N,N-dibenzylhydroxylamine, N,N-diethylhydroxylamine, N,N-dioctylhydroxylamine, N,N-dilaurylhydroxylamine, N,N-ditetradecylhydroxylamine, N,N-dihexadecylhydroxylamine, N,N-diotadecylhydroxylamine, N-hexadecyl-N-octadecylhydroxylamine, N-heptadecyl-N-octadecylhydroxylamine, N,N-dialkylhydroxylamine derived from hydrogenated tallow amine.
7. Nitrones, for example, N-benzyl-alpha-phenyl nitrone, N-ethyl-alpha-methyl nitrone, N-octyl-alpha-heptyl nitrone, N-lauryl-alpha-undecyl nitrone, N-tetradecyl-alpha-tridecyl nitrone, N-hexadecyl-alpha-pentadecyl nitrone, N-octadecyl-alpha-heptadecylnitrone, N-hexadecyl-alpha-heptadecyl nitrone, N-octadecyl-alpha-pentadecyl nitrone, N-heptadecyl-alpha-heptadecyl nitrone, N-octadecyl-alpha-hexadecyl nitrone, nitrone derived from N,N-dialkylhydroxylamine derived from hydrogenated tallow amine.
8. Polyamide stabilisers, for example, copper salts in combination with iodides and/or phosphorus compounds and salts of divalent manganese.
9. Basic co-stabilisers, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example Ca stearate, Zn stearate, Mg behenate, Mg stearate, Na ricinoleate and K palmitate, antimony pyrocatecholate or zinc pyrocatecholate.
10. Nucleating agents, for example, 4-tert.butyl-benzoic acid, adipic acid, diphenylacetic acid.
11. Fillers and reinforcing agents, for example, calcium carbonate, silicates, glass fibres, asbestos, talc, kaolin, mica, barium sulfate, metal oxides and hydroxydes, carbon black, graphite.
12. Other additives, for example, plasticisers, lubricants, emulsifiers, pigments, optical brighteners, flameproofing agents, antistatic agents and blowing agents.

The phenolic antioxidant of particular interest is selected from the group consisting of n-octadecyl 3,5-di-tert-butyl-4-hydroxyhydrocinnamate, neopentanetetrayl tetrakis(3,5-di-tert-butyl-4-hydroxyhydrocinammate), di-n-octadecyl 3,5-di-tert-butyl-4-hydroxybenzylphosphonate,
1,3,5-tris(3,5-di-tert-butyl-4-hydroxybenzyl)isocyanurate, thiodiethylene bis(3,5-di-tert-butyl-4-hydroxyhydrocinnamate), 1,3,5-trimethyl-2,4,6-tris(3,5-di-tert-butyl-4-hydroxybenzyl)benzene, 3,6-dioxaoctamethylene
bis(3-methyl-5-tert-butyl-4-hydroxyhydrocinnamate), 2,6-di-tert-butyl-p-cresol, 2,2'-ethylidene-bis(4,6-di-tert-butylphenol), 1,3,5-tris(2,6-dimethyl-4-tert-butyl-3-hydroxybenzyl)isocynurate,
1,1,3-tris(2-methyl-4-hydroxy-5-tert-butylphenyl)butane, 1,3,5-tris[2-(3,5-di-tert-butyl-4-hydroxyhydrocinnamoyloxy)ethyl]isocyanurate, 3,5-di(3,5-di-tert-butyl-4-hydroxybenzyl)mesitol, hexamethylene
bis(3,5-di-tert-butyl-4-hydroxyhydrocinnamate), 1-(3,5-di-tert-butyl-4-hydroxyanilino)-3,5-di(octylthio)-s-triazine, N,N'-hexamethylene-bis(3,5-di-tert-butyl-4-hydroxyhydrocinnamamide), calcium bis(ethyl 3,5-di-tert-butyl-4-hydroxybenzylphosphonate), ethylene bis[3,3-di(3-tert-butyl-4-hydroxyphenyl)butyrate], octyl 3,5-di-tert-butyl-4-hydroxybenzylmercaptoacetate,
bis(3,5-di-tert-butyl-4-hydroxyhydrocinnamoyl)hydrazide, and
N,N'-bis[2-(3,5-di-tert-butyl-4-hydroxyhydrocinnamoyloxy)-ethyl]-oxamide.

A most preferred phenolic antioxidant is neopentanetetrayl tetrakis(3,5-di-tert-butyl-4-hydroxyhydrocinnamate), n-octadecyl 3,5-di-tert-butyl-4-hydroxyhydrocinnamate,
1,3,5-trimethyl-2,4,6-tris(3,5-di-tert-butyl-4-hydroxybenzyl)benzene, 1,3,5-tris(3,5-di-tert-butyl-4-hydroxybenzyl)isocyanurate, 2,6-di-tert-butyl-p-cresol or 2,2'-ethylidene-bis-(4,6-di-tert-butylphenol).

The hindered amine compound of particular interest is selected from the group consisting of bis(2,2,6,6-tetramethylpiperidin-4-yl)sebacate,
bis(1,2,2,6,6-pentamethylpiperidin-4-yl) sebacate,
di(1,2,2,6,6-pentamethylpiperidin-4-yl) (3,5-di-tert-butyl-4-hydroxybenzyl)butylmalonate, 4-benzoyl-2,2,6,6-tetramethylpiperidine, 4-stearyloxy-2,2,6,6-tetramethylpiperidine, 3-n-octyl-7,7,9,9-tetramethyl-1,3,8-triaza-spiro[4.5]decane-2,4-dione,
tris(2,2,6,6-tetramethylpiperidin-4-yl) nitrilotriacetate,
1,2-bis(2,2,6,6-tetramethyl-3-oxopiperazin-4-yl)ethane, 2,2,4,4-tetramethyl-7-oxa-3,20-diaza-21-oxodispiro[5.1.11.2] heneicosane, polycondensation product of 2,4-dichloro-6-tert-octylamino-s-triazine and
4,4'-hexamethylenebis(amino-2,2,6,6-tetramethylpiperidine), polycondensation product of 1-(2-hydroxyethyl)-2,2,6,6-tetramethyl-4-hydroxypiperidine and succinic acid, polycondensation product of 4,4'-hexamethylenebis-(amino-2,2,6,6-tetramethylpiperidine) and 1,2-dibromoethane,
tetrakis(2,2,6,6-tetramethylpiperidin-4-yl) 1,2,3,4-butanetetracarboxylate, tetrakis(1,2,2,6,6-pentamethylpiperidin-4-yl) 1,2,3,4-butanetetracarboxylate, polycondensation product of 2,4-dichloro-6-morpholino-s-triazine and
4,4'-hexamethylenebis(amino-2,2,6,6-tetramethylpiperidine), N,N',N'',N'''-tetrakis[(4,6-bis(butyl-2,2,6,6-tetramethyl-piperidin-4-yl)-amino-s-triazin-2-yl]-1,10-diamino-4,7-diaza decane, mixed
[2,2,6,6-tetramethylpiperidin-4-yl/β,β,β',β'-tetramethyl-3,9-(2,4,8,10-tetraoxaspiro[5.5]undecane) diethyl] 1,2,3,4-butanetetracarboxylate,
mixed
[1,2,2,6,6-pentamethylpiperidin-4-yl/β,β,β',β'-tetramethyl-3,9-(2,4,8,10-tetraoxaspiro[5.5] undecane)diethyl] 1,2,3,4-butanetetracarboxylate, octamethylene bis(2,2,6,6-tetramethylpiperidin-4-carboxylate), 4,4'-ethylenebis(2,2,6,6-tetramethlypiperazin-3-one) and bis(1-octyloxy-2,2,6,6-tetramethylpiperidin-4-yl) sebacate.

A most preferred hindered amine compound is bis(2,2,6,6-tetramethylpiperidin-4-yl) sebacate, the polycondensation product of 1-(2-hydroxyethyl)-2,2,6,6-tetramethyl-4-hydroxypiperdine and succinic acid, the polycondensation product of
2,4-dichloro-6-tert-octylamino-s-triazine and
4,4'-hexamethylenebis(amino-2,2,6,6-tetramethylpiperidine), N,N',N'',N'''-tetrakis[(4,6-bis(butyl-(2,2,6,6-tetramethyl-piperidin-4-yl)amino)-s-triazine-2-yl]-1,10-diamino-4,7-diazadecane or bis(1-octyloxy-2,2,6,6-tetramethylpiperidin-4-yl) sebacate.

The following examples are presented for the purpose of illustration only and are not to be construed as limiting the nature or scope of the instant invention in any manner whatsoever. Percentages are by weight if not otherwise indicated.

### Example 1: 1,4-Bis(2,6-diphenylpiperidin-1-yl)-2-butene

A solution of 2,6-diphenylpiperidine [3.0 g, 12.6 mmol; prepared by the method of V. Baliah et al., Indian J. Chem., 16B, 1965 (1978)] and 1,4-but-2-enediol diacetate (1.1 g, 6.3 mmol) in tetrahydrofuran (20 ml, THF) is treated with tetrakis(triphenylphosphine)palladium (0) (0.65 g, 0.6 mmol) and stirred at room temperature overnight. The reaction mixture is concentrated in vacuo to a solid that is redissolved in THF (100 ml) and treated with 20% aqueous sodium hydroxide (25 ml). The mixture is stirred for one hour, concentrated, and extracted with diethyl ether. The ether extracts are dried and concentrated giving an orange solid which is purified by crystallization from methanol to yield 2.02 g (61 %) of the title compound as a white solid melting at 197-200°C.

| Analysis: | | | |
|---|---|---|---|
| Calcd for C₃₈H₄₂N₂: | C, 86.6; | H, 8.0; | N, 5.3. |
| Found: | C, 86.7; | H, 8.0; | N, 5.2. |

### Example 2: 1-(2,6-Diphenylpiperidin-1-yl)-4-dibenzylamino-2-butene

A solution of dibenzylamine (40 g, 0.20 mol) and 1,4-but-2-enediol diacetate (105 g, 0.61 mol) in THF (150 ml) is treated with tetrakis(triphenylphosphine)palladium (0) (1 g, 0.9 mmol) and stirred at room temperature overnight. The reaction mixture is concentrated in vacuo and the excess 1,4-but-2-enediol is removed by bulb-to-bulb distillation (80-90°C, 1mm). The residue is purified by eluting through a short plug of silica gel (1:1, ethyl acetate:hexanes). 59.2 g (95%)of 1-acetoxy-4-dibenzylamino-2-butene is obtained.

A solution of 1-acetoxy-4-dibenzylamino-2-butene (3.0 g, 9.8 mmol) and 2,6-diphenylpiperidine (2.3 g, 9.8 mmol) in THF (25 ml) is treated with tetrakis(triphenylphosphine)palladium (0) (0.5 g, 0.45 mmol) and stirred at room temperature overnight. The reaction mixture is concentrated in vacuo. The residue is redissolved in THF (100 ml) and treated with 20% aqueous sodium hydroxide (25 ml). The mixture is stirred for one hour, concentrated, and extracted with diethyl ether. The ether extracts are dried and concentrated to give an orange oil which is purified by chromatography (silica gel; 5% ethyl acetate in hexanes) to give the title compound in a yield of 2.8 g (59%) as a clear oil; MS m/z 486 (M⁺).

### Example 3: Process Stabilization of Dynamically Crosslinked Polypropylene/Nitrile Rubber

To a Brabender Plasticorder heated to 190°C is added 55 grams of dynamically crosslinked polypropylene/nitrile rubber (GEOLAST, Monsanto). The polymer is mixed under nitrogen for 3 minutes at 30 rpm and then 2.2 grams (4% by weight of the resin blend) of the test compound is added under nitrogen and mixed into the resin blend at 90 rpm for 7 minutes. The test sample is then removed from the Brabender and flattened in a cold press.

The test sample is then compression molded into plaques (4" x4" x 60 mils; 10.16 cm x 10.16 cm x 1.524 mm) at 200°C for 4 minutes at 2000 psi (140 Kg/cm²) and then 4 minutes at 50,000 psi (3500 Kg/cm²).

The plaques are then cut into tensile bars and oven aged in a forced draft oven at 135°C for 7 days. The aged samples are tested for % retention of elongation according to ASTM D412.

The instant compounds of Examples 1 and 2 provide the GEOLAST resin with effective stabilization against thermal oxidative degradation.

## Claims

1. A compound of formula I or formula II
T-CH₂-CH=CH-CH₂-T (I)
T-CH₂-CH=CH-CH₂-N(E₁)(E₂) (II)
wherein
E₁ and E₂ are independently alkyl of 1 to 30 carbon atoms, said alkyl terminated with cycloalkyl of 5 to 12 carbon atoms, said alkyl terminated with -CN, -OR₁₆, -NR₁₇R₁₈, -SR₁₉, -COOR₂₀ or -CONR₂₁R₂₂, where R₁₆, R₁₇, R₁₈, R₁₉ and R₂₀ are independently alkyl of 1 to 20 carbon atoms or alkenyl of 3 to 18 carbon atoms, and R₂₁ and R₂₂ are independently hydrogen or the same meaning as R₁₆; or said alkyl interrupted by one or more -O-, -S-, -SO-, -SO₂-, -CO-, -COO-, -OCO-, -CONR₂₃-, -NR₂₃CO- or -NR₂₄- where R₂₃ and R₂₄ have the same meaning as R₂₁; alkenyl of 3 to 20 carbon atoms; aralkyl of 7 to 15 carbon atoms or said aralkyl substituted on the aryl ring by one to three groups selected from alkyl of 1 to 12 carbon atoms, -CN, -NO₂, halogen, -OR₁₆, -NR₁₇R₁₈, -SR₁₉, -COOR₂₀ or -CONR₂₁R₂₂; or aryl of 6 to 10 carbon atoms or said aryl substituted by one to three substituents selected from the group consisting of alkyl of 1 to 20 carbon atoms, cycloalkyl of 5 to 12 carbon atoms and aralkyl of 7 to 15 carbon atoms;
T is a group of formula III, IV, V or VI 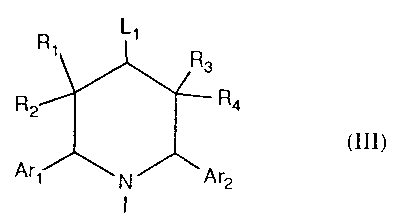 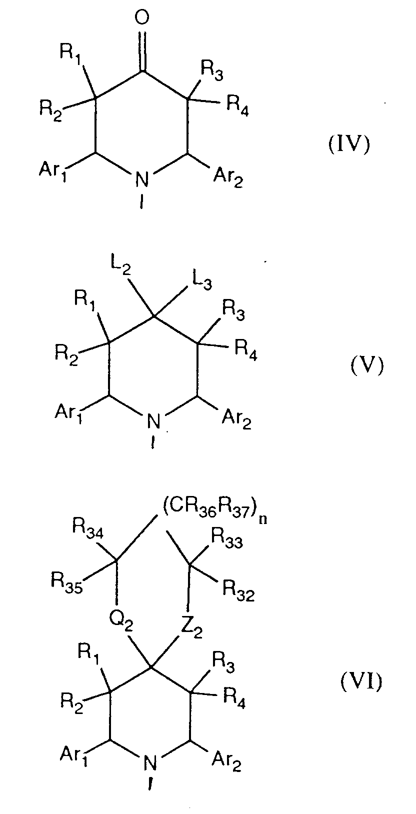 where Ar₁ and Ar₂ are independently aryl of 6 to 10 carbon atoms; or said aryl substituted by one to three substituents selected from the group consisting of alkyl of 1 to 20 carbon atoms, cycloalkyl of 5 to 12 carbon atoms, phenylalkyl of 7 to 15 carbon atoms, -COOG₁ where G₁ is hydrogen or alkyl of 1 to 20 carbon atoms, -COG₂ where G₂ is alkyl of 1 to 20 carbon atoms, -NR₅R₆ where R₅ and R₆ are independently hydrogen or alkyl of 1 to 20 carbon atoms, -SG₃ where G₃ is aryl of 6 to 10 carbon atoms or alkyl of 1 to 20 carbon atoms, -OH, -OCH₃, -CN, -CF₃, -NO₂, -F, -Cl, -Br or -I;
R₁, R₂, R₃ and R₄ are independently hydrogen; a linear or branched alkyl of 1 to 30 carbon atoms; said alkyl terminated with -OR₇, -NR₈R₉, -SR₁₀, -COOR₁₁ or -CONR₁₂R₁₃, where R₇, R₈, R₉, R₁₀ and R₁₁ are independently hydrogen, alkyl of 1 to 20 carbon atoms or alkenyl of 3 to 18 carbon atoms, and R₁₂ and R₁₃ are independently hydrogen or the same meaning as R₁₁; or said alkyl interrupted by one or more -O-, -S-, -SO-, -SO₂-, -CO-, -COO-, -OCO-, -CONR₁₄-, -NR₁₄CO- or -NR₁₅- where R₁₄ and R₁₅ have the same meaning as R₁₂; alkenyl of 3 to 20 carbon atoms; or aryl of 6 to 10 carbon atoms;
L₁ is hydrogen, -OR₂₅ where R₂₅ is hydrogen or alkyl of 1 to 30 carbon atoms, -NR₂₆R₂₇, -OCOR₂₈, -NCOR₂₉ where R₂₆, R₂₇, R₂₈ and R₂₉ have the same meaning as R₂₅;
L₂ and L₃ are independently -Q₁-R₃₀ or -Z₁-R₃₁ where Q₁ and Z₁ are independently -O-, -S- or -NR₂₆-, and R₃₀ and R₃₁ are hydrogen or alkyl of 1 to 30 carbon atoms; and
n is 0 or 1, Q₂ and Z₂ are independently -O-, -S- or -NR₂₆-, and R₃₂, R₃₃, R₃₄, R₃₅, R₃₆ and R₃₇ are independently hydrogen or alkyl of 1 to 30 carbon atoms.

2. A compound according to claim 1 wherein
E₁ and E₂ are independently alkyl of 4 to 20 carbon atoms or said alkyl interrupted by one or more -O-, -S-, -CO-, -COO-, -OCO-, -CONR₂₃-, -NR₂₃CO- or -NR₂₄- where R₂₃ and R₂₄ are as defined in claim 1; alkenyl of 3 to 12 carbon atoms; phenylalkyl of 7 to 15 carbon atoms or said phenylalkyl substituted on the phenyl ring by one to three groups selected from alkyl of 1 to 12 carbon atoms, -OR₁₆, -NR₁₇R₁₈, -COOR₂₀ or -CONR₂₁R₂₂; or phenyl or said phenyl substituted by one to three substituents selected from the group consisting of alkyl of 1 to 20 carbon atoms and cycloalkyl of 5 to 7 carbon atoms;
T is a group of formula III or IV;
Ar₁ and Ar₂ are independently phenyl; or said phenyl substituted by one to three substituents selected from the group consisting of alkyl of 1 to 20 carbon atoms, cycloalkyl of 5 to 7 carbon atoms, phenylalkyl of 7 to 15 carbon atoms, -OH, -OCH₃, -COOG₁, -COG₂ or -NR₅R₆, where G₁, G₂, R₅ and R₆ are as defined in claim 1; R₁, R₂, R₃ and R₄ are independently hydrogen, methyl, ethyl, -CH₂-OH, -CH₂-NH₂, -C₂H₄-OH, -C₂H₄-NH₂, allyl or phenyl; and
L₁ is as defined in claim 1.

3. A compound according to claim 2 wherein
E₁ and E₂ are independently alkyl of 4 to 20 carbon atoms; phenylalkyl of 7 to 15 carbon atoms; or said phenylalkyl substituted on the phenyl ring by one or two alkyl groups of 1 to 4 carbon atoms and/or -OH; or phenyl, tolyl or xylyl;
Ar₁ and Ar₂ are independently phenyl; or said phenyl substituted by cyclohexyl, -OH, -OCH₃ and/or one or two alkyl groups of 1 to 4 carbon atoms; and
L₁ is hydrogen or -OH.

4. A compound according to claim 3 wherein Ar₁ and Ar₂ are phenyl or phenyl substituted by one or two methyl groups.

5. A compound according to claim 3 wherein R₁, R₂, R₃ and R₄ are independently hydrogen or methyl.

6. A compound according to claim 3 wherein E₁ and E₂ are independently alkyl of 4 to 20 carbon atoms or benzyl.

7. A compound according to claim 3 wherein T is a structure of formula III where L₁ is hydrogen.

8. A composition stabilized against the deleterious effects of oxygen, heat or actinic light which comprises
(a) an organic material subject to oxidative, thermal or actinic degradation, and
(b) an effective stabilizing amount of a compound of formula I or II according to claim 1.

9. A composition according to claim 8 wherein component (a) is a synthetic polymer.

10. A composition according to claim 9 wherein the synthetic polymer is dynamically crosslinked polypropylene/nitrile rubber.

11. A composition according to claim 8 comprising in addition to components (a) and (b) one or more conventional stabilizers or conventional additives.

12. Use of a compound of formula I or II according to claim 1 as stabilizer for protecting organic material against oxidative, thermal or actinic degradation.

13. Use according to claim 12 for protecting a polyolefin or an elastomer against degradation during processing at elevated temperatures.

14. A process for stabilizing an organic material against oxidative, thermal or actinic degradation, which process comprises incorporating in said material at least one compound of formula I or II according to claim 1.

## Patentansprüche

1. Verbindungen der Formel I oder II
T-CH₂-CH=CH-CH₂-T (I)
T-CH₂-CH=CH-CH₂-N(E₁)(E₂) (II)
worin
E₁ und E₂ unabhängig Alkyl mit 1 bis 30 Kohlenstoffatomen, dieses Alkyl terminiert mit Cycloalkyl mit 5 bis 12 Kohlenstoffatomen, dieses Alkyl terminiert mit -CN, -OR₁₆, NR₁₇R₁₈, -SR₁₉, -COOR₂₀ oder -CONR₂₁R₂₂,worin R₁₆, R₁₇, R₁₈, R₁₉ und R₂₀ unabhängig Alkyl mit 1 bis 20 Kohlenstoffatomen oder Alkenyl mit 3 bis 18 Kohlenstoffatomen bedeuten und R₂₁ und R₂₂ unabhängig Wasserstoff bedeuten oder die gleiche Bedeutung wie R₁₆ besitzen; oder dieses Alkyl unterbrochen durch ein oder mehrere -O-, -S-, -SO-, -SO₂-, -CO-, -COO-, -OCO-, -CONR₂₃-, -NR₂₃CO- oder -NR₂₄-, worin R₂₃ und R₂₄ die gleiche Bedeutung wie R₂₁ besitzen; Alkenyl mit 3 bis 20 Kohlenstoffatomen; Aralkyl mit 7 bis 15 Kohlenstoffatomen oder dieses Aralkyl substituiert an dem Arylring durch ein bis drei Gruppen, ausgewählt unter Alkyl mit 1 bis 12 Kohlenstoffatomen, -CN, -NO₂, Halogen, -OR₁₆, -NR₁₇R₁₈, -SR₁₉, -COOR₂₀ oder -CONR₂₁R₂₂; oder Aryl mit 6 bis 10 Kohlenstoffatomen oder dieses Aryl substituiert durch ein bis drei Substituenten, ausgewählt unter Alkyl mit 1 bis 20 Kohlenstoffatomen, Cycloalkyl mit 5 bis 12 Kohlenstoffatomen und Aralkyl mit 7 bis 15 Kohlenstoffatomen, wiedergeben;
T für eine Gruppe der Formel III, IV, V oder VI steht 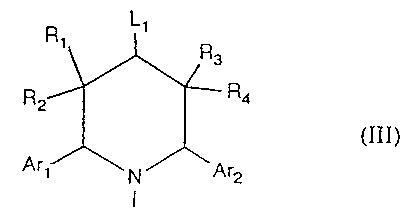 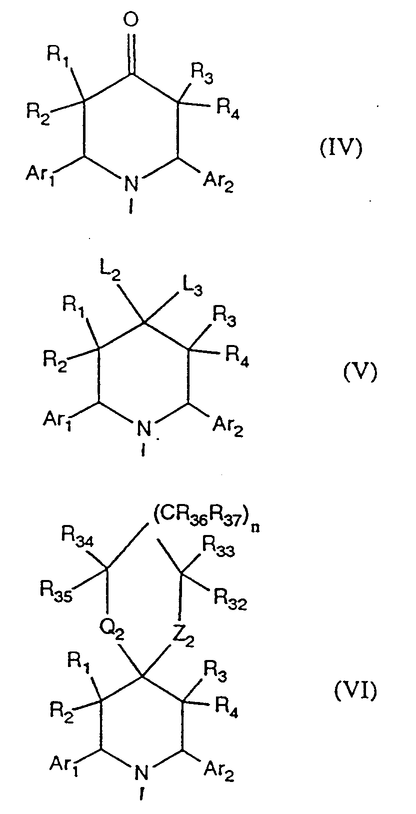 worin Ar₁ und Ar₂ unabhängig Aryl mit 6 bis 10 Kohlenstoffatomen; oder dieses Aryl substituiert durch ein bis drei Substituenten, ausgewählt unter Alkyl mit 1 bis 20 Kohlenstoffatomen, Cycloalkyl mit 5 bis 12 Kohlenstoffatomen, Phenylalkyl mit 7 bis 15 Kohlenstoffatomen, -COOG₁, worin G₁ für Wasserstoff oder Alkyl mit 1 bis 20 Kohlenstoffatomen steht, -COG₂, worin G₂ für Alkyl mit 1 bis 20 Kohlenstoffatomen steht, -NR₅R₆, worin R₅ und R₆ unabhängig Wasserstoff oder Alkyl mit 1 bis 20 Kohlenstoffatomen darstellen, -SG₃, worin G₃ für Aryl mit 6 bis 10 Kohlenstoffatomen oder Alkyl mit 1 bis 20 Kohlenstoffatomen steht, -OH, -OCH₃, -CN, -CF₃, -NO₂, -F, -Cl, -Br oder -J bedeuten;
R₁, R₂, R₃ und R₄ unabhängig Wasserstoff; lineares oder verzweigtes Alkyl mit 1 bis 30 Kohlenstoffatomen; dieses Alkyl terminiert mit -OR₇, -NR₈R₉, -SR₁₀, -COOR₁₁ oder -CONR₁₂R₁₃, worin R₇, R₈, R₉, R₁₀ und R₁₁ unabhängig Wasserstoff, Alkyl mit 1 bis 20 Kohlenstoffatomen oder Alkenyl mit 3 bis 18 Kohlenstoffatomen wiedergeben und R₁₂ und R₁₃ unabhängig Wasserstoff darstellen oder die gleiche Bedeutung wie R₁₁ besitzen; oder dieses Alkyl unterbrochen durch ein oder mehrere -O-, -S-, -SO-, -SO₂-, -CO-, -COO-, -OCO-, -CONR₁₄-, -NR₁₄CO- oder -NR₁₅-, worin R₁₄ und R₁₅ die gleiche Bedeutung wie R₁₂ besitzen; Alkenyl mit 3 bis 20 Kohlenstoffatomen; oder Aryl mit 6 bis 10 Kohlenstoffatomen bedeuten;
L₁ für Wasserstoff, -OR₂₅, worin R₂₅ Wasserstoff oder Alkyl mit 1 bis 30 Kohlenstoffatomen bedeutet, -NR₂₆R₂₇, -OCOR₂₈, -NCOR₂₉, worin R₂₆, R₂₇, R₂₈ und R₂₉ die gleiche Bedeutung wie R₂₅ besitzen, steht;
L₂ und L₃ unabhängig für -Q₁-R₃₀ oder -Z₁-R₃₁ stehen, worin Q₁ und Z₁ unabhängig -O-, -S- oder -NR₂₆- bedeuten und R₃₀ und R₃₁ Wasserstoff oder Alkyl mit 1 bis 30 Kohlenstoffatomen wiedergeben; und
n für 0 oder 1 steht, Q₂ und Z₂ unabhängig für -O-, -S- oder -NR₂₆- stehen und R₃₂, R₃₃, R₃₄, R₃₅, R₃₆ und R₃₇ unabhängig Wasserstoff oder Alkyl mit 1 bis 30 Kohlenstoffatomen bedeuten.

2. Verbindung gemäß Anspruch 1, worin E₁ und E₂ unabhängig Alkyl mit 4 bis 20 Kohlenstoffatomen oder dieses Alkyl unterbrochen durch ein oder mehrere -O-, -S-, -CO-, -COO-, -OCO-, -CONR₂₃-, -NR₂₃CO- oder -NR₂₄-, worin R₂₃ und R₂₄ wie in Anspruch 1 definiert sind; Alkenyl mit 3 bis 12 Kohlenstoffatomen; Phenylalkyl mit 7 bis 15 Kohlenstoffatomen oder dieses Phenylalkyl substituiert an dem Phenylring durch ein bis drei Gruppen, ausgewählt unter Alkyl mit 1 bis 12 Kohlenstoffatomen, -OR₁₆, -NR₁₇R₁₈, -COOR₂₀ oder -CONR₂₁R₂₂; oder Phenyl oder dieses Phenyl substituiert durch ein bis drei Substituenten, ausgewählt unter Alkyl mit 1 bis 20 Kohlenstoffatomen und Cycloalkyl mit 5 bis 7 Kohlenstoffatomen, bedeuten;
T für eine Gruppe der Formel III oder IV steht;
Ar₁ und Ar₂ unabhängig Phenyl; oder dieses Phenyl substituiert durch ein bis drei Substituenten, ausgewählt unter Alkyl mit 1 bis 20 Kohlenstoffatomen, Cycloalkyl mit 5 bis 7 Kohlenstoffatomen, Phenylalkyl mit 7 bis 15 Kohlenstoffatomen, -OH, -OCH₃, -COOG₁, -COG₂ oder NR₅R₆, worin G₁, G₂, R₅ und R₆ wie in Anspruch 1 definiert sind, bedeuten;
R₁, R₂, R₃ und R₄ unabhängig Wasserstoff, Methyl, Ethyl, -CH₂-OH, -CH₂-NH₂, -C₂H₄-OH, -C₂H₄-NH₂, Allyl oder Phenyl bedeuten; und
L₁ wie in Anspruch 1 definiert ist.

3. Verbindung gemäß Anspruch 2, worin
E₁ und E₂ unabhängig für Alkyl mit 4 bis 20 Kohlenstoffatomen; Phenylalkyl mit 7 bis 15 Kohlenstoffatomen oder dieses Phenylalkyl substituiert an dem Phenylring durch ein oder zwei Alkylgruppen mit 1 bis 4 Kohlenstoffatomen und/oder -OH; oder Phenyl, Tolyl oder Xylyl stehen;
Ar₁ und Ar₂ unabhängig Phenyl oder dieses Phenyl substituiert durch Cyclohexyl, -OH, -OCH₃ und/oder ein oder zwei Alkylgruppen mit 1 bis 4 Kohlenstoffatomen bedeuten; und L₁ für Wasserstoff oder -OH steht.

4. Verbindung gemäß Anspruch 3, worin Ar₁ und Ar₂ Phenyl oder Phenyl substituiert durch ein oder zwei Methylgruppen bedeuten.

5. Verbindung gemäß Anspruch 3, worin R₁, R₂, R₃ und R₄ unabhängig Wasserstoff oder Methyl bedeuten.

6. Verbindung gemäß Anspruch 3, worin E₁ und E₂ unabhängig Alkyl mit 4 bis 20 Kohlenstoffatomen oder Benzyl wiedergeben.

7. Verbindung gemäß Anspruch 3, Worin T eine Struktur der Formel III besitzt, in der L₁ für Wasserstoff steht.

8. Gegenüber schädlichen Wirkungen infolge von Sauerstoff, Wärme oder aktinischem Licht stabilisierte Zusammensetzung, die
(a) ein organisches Material, welches einem oxidativen, thermischen oder aktinischen Abbau unterliegt, und
(b) eine wirksame, stabilisierende Menge einer Verbindung der Formel I oder II gemäß Anspruch 1 umfaßt.

9. Zusammensetzung gemäß Anspruch 8, worin die Komponente (a) ein synthetisches Polymeres ist.

10. Zusammensetzung gemäß Anspruch 9, worin das synthetische Polymere dynamisch vernetzter Polypropylen/Nitrilkautschuk ist.

11. Zusammensetzung gemäß Anspruch 8, die zusätzlich zu den Komponenten (a) und (b) einen oder mehrere übliche Stabilisatoren oder übliche Additive umfaßt.

12. Verwendung einer Verbindung der Formel I oder II gemäß Anspruch 1 als Stabilisator zum Schutz von organischen Materialien gegenüber oxidativem, thermischem oder aktinischem Abbau.

13. Verwendung gemäß Anspruch 12 zum Schutz eines Polyolefins oder eines Elastomeren gegenüber dem Abbau während der Verarbeitung bei erhöhten Temperaturen.

14. Verfahren zur Stabilisierung eines organischen Materials gegenüber oxidativem, thermischem oder aktinischem Abbau, das das Einbringen zumindest einer Verbindung der Formel I oder II gemäß Anspruch 1 in dieses Material umfaßt.

## Revendications

1. Un composé de formule I ou II
T-CH₂-CH=CH-CH₂-T (I)
T-CH₂-CH=CH-CH₂-N(E₁)(E₂) (II)
où
E₁ et E₂ sont chacun indépendamment un groupe alkyle de 1 à 30 atomes de carbone, un tel groupe alkyle terminé par un groupe cycloalkyle de 5 à 12 atomes de carbone, un tel groupe alkyle terminé par -CN, -OR₁₆, -NR₁₇R₁₈, -SR₁₉, -COOR₂₀ ou -CONR₂₁R₂₂, où R₁₆, R₁₇, R₁₈, R₁₉ et R₂₀ sont chacun indépendamment un groupe alkyle de 1 à 20 atomes de carbone ou alcényle de 3 à 18 atomes de carbone et R₂₁ et R₂₂ sont chacun indépendamment l'hydrogène ou un groupe tel que défini pour R₁₆, ou bien un tel groupe alkyle qui est interrompu par un ou plusieurs -O-, -S-, -SO-, -SO₂-, -CO-, -COO-, -OCO-, -CONR₂₃-, -NR₂₃CO- ou -NR₂₄- où R₂₃ et R₂₄ ont la même définition que R₂₁ ; un groupe alcényle de 3 à 20 atomes de carbone ; un groupe aralkyle de 7 à 15 atomes de carbone ou un tel groupe aralkyle substitué sur le noyau arylique par un à trois groupes choisis parmi les groupes alkyle de 1 à 12 atomes de carbone, -CN, -NO₂, les halogènes, -OR₁₆, -NR₁₇R₁₈, -SR₁₉, -COOR₂₀ ou -CONR₂₁R₂₂ ; ou un groupe aryle de 6 à 10 atomes de carbone, ou un tel groupe aryle substitué par un à trois substituants choisis parmi les groupes alkyle de 1 à 20 atomes de carbone, cycloalkyle de 5 à 12 atomes de carbone et aralkyle de 7 à 15 atomes de carbone ;
T est un groupe de formule III, IV, V ou VI 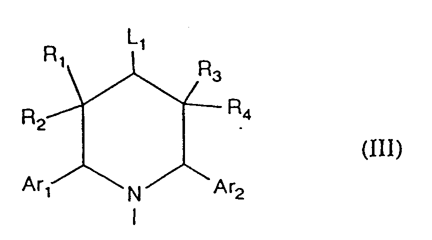 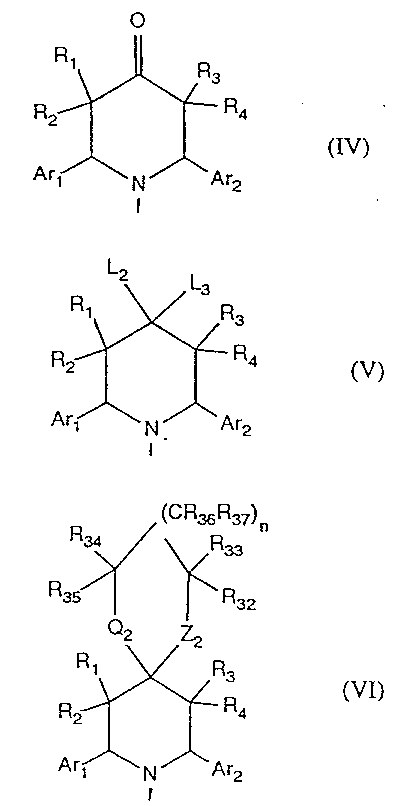 où Ar₁ et Ar₂ sont chacun indépendamment un groupe aryle de 6 à 10 atomes de carbone, ou un tel groupe aryle substitué par un à trois substituants choisis parmi les groupes alkyle de 1 à 20 atomes de carbone, cycloalkyle de 5 à 12 atomes de carbone, phénylalkyle de 7 à 15 atomes de carbone, -COOG₁ où G₁ est l'hydrogène ou un groupe alkyle de 1 à 20 atomes de carbone, -COG₂ où G₂ est un groupe alkyle de 1 à 20 atomes de carbone, -NR₅R₆ où R₅ et R₆ sont chacun indépendamment l'hydrogène ou un groupe alkyle de 1 à 20 atomes de carbone, -SG₃ où G₃ est un groupe aryle de 6 à 10 atomes de carbone ou alkyle de 1 à 20 atomes de carbone, -OH, -OCH₃, -CN, -CF₃, -NO₂, -F, -Cl, -Br ou -I ;
R₁, R₂, R₃ et R₄ sont chacun indépendamment l'hydrogène ; un groupe alkyle linéaire ou ramifié de 1 à 30 atomes de carbone, un tel groupe alkyle terminé par -OR₇, -NR₈R₉, -SR₁₀, -COOR₁₁ ou -CONR₁₂R₁₃ où R₇, R₈, R₉, R₁₀ et R₁₁ sont chacun indépendamment l'hydrogène, un groupe alkyle de 1 à 20 atomes de carbone ou alcényle de 3 à 18 atomes de carbone et R₁₂ et R₁₃ sont chacun indépendamment l'hydrogène ou un groupe tel que défini pour R₁₁, ou un tel groupe alkyle interrompu par un ou plusieurs -O-, -S-, -SO-, -SO₂-, -CO-, -COO-, -OCO-, -CONR₁₄-, -NR₁₄CO- ou -NR₁₅- où R₁₄ et R₁₅ ont la même définition que R₁₂ ; un groupe alcényle de 3 à 20 atomes de carbone ; ou un groupe aryle de 6 à 10 atomes de carbone ;
L₁ est l'hydrogène, -OR₂₅ ou R₂₅ est l'hydrogène ou un groupe alkyle de 1 à 30 atomes de carbone, -NR₂₆R₂₇, -OCOR₂₈, -NCOR₂₉ où R₂₆, R₂₇, R₂₈ et R₂₉ ont la même définition que R₂₅ ;
L₂ et L₃ sont chacun indépendamment -Q₁-R₃₀ ou -Z₁-R₃₁ où Q₁ et Z₁ sont chacun indépendamment -O-, -S- ou -NR₂₆- et R₃₀ et R₃₁ sont chacun l'hydrogène ou un groupe alkyle de 1 à 30 atomes de carbone ; et
n est 0 ou 1, Q₂ et Z₂ sont chacun indépendamment -O-, -S- ou -NR₂₆-, et R₃₂, R₃₃, R₃₄, R₃₅, R₃₆ et R₃₇ sont chacun indépendamment l'hydrogène ou un groupe alkyle de 1 à 30 atomes de carbone.

2. Un composé selon la revendication 1, dans lequel
E₁ et E₂ sont chacun indépendamment un groupe alkyle de 4 à 20 atomes de carbone ou un tel groupe alkyle interrompu par un ou plusieurs -O-, -S-, -CO-, -COO-, -OCO-, -CONR₂₃-, -NR₂₃CO- ou -NR₂₄-, où R₂₃ et R₂₄ sont tels que définis dans la revendication 1 ; un groupe alcényle de 3 à 12 atomes de carbone ; un groupe phénylalkyle de 7 à 15 atomes de carbone ou un tel groupe phénylalkyle substitué sur le noyau phénylique par un à trois groupes choisis parmi les groupes alkyle de 1 à 12 atomes de carbone, -OR₁₆, -NR₁₇R₁₈, -COOR₂₀ ou -CONR₂₁R₂₂ ; ou un groupe phényle ou ledit groupe phényle substitué par un à trois substituants choisis parmi les groupes alkyle de 1 à 20 atomes de carbone et cycloalkyle de 5 à 7 atomes de carbone ;
T est un groupe de formule III ou IV ;
Ar₁ et Ar₂ sont chacun indépendamment un groupe phényle ou ledit groupe phényle substitué par un à trois substituants choisis parmi les groupes alkyle de 1 à 20 atomes de carbone, cycloalkyle de 5 à 7 atomes de carbone, phénylalkyle de 7 à 15 atomes de carbone, -OH, -OCH₃, -COOG₁, -COG₂ ou -NR₅R₆, où G₁, G₂, R₅ et R₆ sont tels que définis dans la revendication 1 ;
R₁, R₂, R₃ et R₄ sont chacun indépendamment l'hydrogène, un groupe méthyle, un groupe éthyle, -CH₂-OH, -CH₂-NH₂, -C₂H₄-OH, -C₂H₄-NH₂, un groupe allyle ou un groupe phényle ; et
L₁ est tel que défini dans la revendication 1.

3. Un composé selon la revendication 2, dans lequel
E₁ et E₂ sont chacun indépendamment un groupe alkyle de 4 à 20 atomes de carbone ; un groupe phénylalkyle de 7 à 15 atomes de carbone ou un tel groupe phénylalkyle substitué sur le noyau phénylique par un ou deux groupes alkyle de 1 à 4 atomes de carbone et/ou -OH ; ou un groupe phényle, tolyle ou xylyle ;
Ar₁ et Ar₂ sont chacun indépendamment un groupe phényle ou ledit groupe phényle substitué par un groupe cyclohexyle, -OH, -OCH₃ et/ou un ou deux groupes alkyle de 1 à 4 atomes de carbone ; et
L₁ est l'hydrogène ou -OH.

4. Un composé selon la revendication 3, dans lequel Ar₁ et Ar₂ sont chacun un groupe phényle ou phényle substitué par un ou deux groupes méthyle.

5. Un composé selon la revendication 3, dans lequel R₁, R₂, R₃ et R₄ sont chacun indépendamment l'hydrogène ou un groupe méthyle.

6. Un composé selon la revendication 3, dans lequel E₁ et E₂ sont chacun indépendamment un groupe alkyle de 4 à 20 atomes de carbone ou benzyle.

7. Un composé selon la revendication 3, dans lequel T est une structure de formule III où L₁ est l'hydrogène.

8. Une composition stabilisée contre les effets nuisibles de l'oxygène, de la chaleur ou de la lumière actinique, qui comprend
(a) une matière organique sensible à une dégradation par l'oxydation, la chaleur ou la lumière actinique, et
(b) une quantité stabilisante efficace d'un composé de formule I ou II selon la revendication 1.

9. Une composition selon la revendication 8, dans laquelle le composant (a) est un polymère synthétique.

10. Une composition selon la revendication 9, dans laquelle le polymère synthétique est un caoutchouc polypropylène/nitrile réticulé dynamiquement.

11. Une composition selon la revendication 8, comprenant, en plus des composants (a) et (b), un ou plusieurs stabilisants classiques ou additifs classiques.

12. Utilisation d'un composé de formule I ou II selon la revendication 1, comme stabilisant pour protéger une matière organique contre une dégradation par l'oxydation, la chaleur ou la lumière actinique.

13. Utilisation selon la revendication 12 pour protéger une polyoléfine ou un élastomère contre une dégradation pendant la mise en oeuvre à des températures élevées.

14. Un procédé pour stabiliser une matière organique contre une dégradation par l'oxydation, la chaleur ou la lumière actinique, lequel procédé consiste à incorporer à ladite matière au moins un composé de formule I ou II selon la revendication 1.
